(19) Europäisches Patentamt
European Patent Office
Office européen des brevets

(11) Veröffentlichungsnummer: **0 025 883**
**B1**

# (12) EUROPÄISCHE PATENTSCHRIFT

(45) Veröffentlichungstag der Patentschrift: **12.01.83**

(51) Int. Cl.³: **C 25 B 3/02,** C 07 C 41/50, C 07 C 43/315, C 07 C 47/565

(21) Anmeldenummer: **80104976.8**

(22) Anmeldetag: **21.08.80**

(54) **Elektrochemisches Verfahren zur Herstellung von 4-tert.-Butoxybenzaldehyddialkylacetalen, ihre Verwendung und 4-tert.-Butoxybenzaldehyddimethylacetal.**

(30) Priorität: **01.09.79 DE 2935398**

(43) Veröffentlichungstag der Anmeldung: **01.04.81 Patentblatt 81/13**

(45) Bekanntmachung des Hinweises auf die Patenterteilung: **12.01.83 Patentblatt 83/2**

(84) Benannte Vertragsstaaten: **DE FR GB IT**

(56) Entgegenhaltungen:
**Chemical Abstracts Band 82, Nr. 11, 17. März 1975, Columbus, Ohio, USA, W. R. YOUNG et al. "Mesomorphic substances containing some Group IV elements", Seite 390, Spalte 2, Abstract Nr. 72453r in Verbindung mit Chemical Substance Index 1972 bis 1976, Seite 3697CS, Spalte 2, Zeilen 111 bis 112**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Barl, Manfred, Dr., Pappelstrasse 2, D-6701 Otterstadt (DE)**
Erfinder: **Degner, Dieter, Dr., Kurpfalzstrasse 8, D-6701 Dannstadt-Schauernheim (DE)**
Erfinder: **Siegel, Hardo, Dr., Hans-Purrmann-Allee 25, D-6720 Speyer (DE)**
Erfinder: **Hoffmann, Werner, Dr., Ringstrasse 11 C, D-6701 Neuhofen (DE)**

EP 0 025 883 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Elektrochemisches Verfahren zur Herstellung von 4-tert.-Butoxybenzaldehyddialkylacetalen, ihre Verwendung und 4-tert.-Butoxybenzaldehyddimethylacetal

Diese Erfindung betrifft ein elektrochemisches Verfahren zur Herstellung von 4-tert.-Butoxybenzaldehyddialkylacetalen, die Verwendung dieser Acetale für die Herstellung von 4-Hydroxybenzaldehyd und das neue 4-tert.-Butoxybenzaldehyddimethylacetal.

Nach dem Verfahren der Erfindung werden die 4-tert.-Butoxybenzaldehyddialkylacetale der Formel

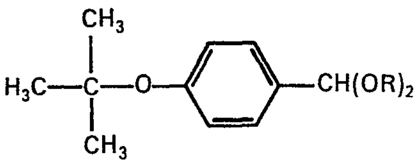

in der R eine Alkylgruppe mit 1 bis 8 C-Atomen bedeutet, dadurch hergestellt, dass man 4-tert.-Butoxytoluol in Gegenwart eines Alkohols der Formel ROH, in der R die oben genannte Bedeutung hat, elektrochemisch oxidiert. Als Alkohole der Formel ROH kommen vorzugsweise Methanol, Äthanol, n-Propanol und n- bzw. iso-Butanol in Betracht.

Von den erfindungsgemäss erhältlichen Verbindungen ist das noch nicht beschriebene 4-tert.-Butoxybenzaldehyddimethylacetal von besonderem technischen Interesse.

In Journal of the Chemical Society Perkin Transactions I, 1978, Seiten 708-715 wird die anodische Oxidation von Alkylbenzolen mit paraständigen Methoxygruppen in Gegenwart von Methanol beschrieben. Dabei wird aus p-Methoxytoluol p-Methoxybenzaldehyddimethylacetal erhalten. Da bei anodischen Oxidationen an der Anode Protonen gebildet werden, werden bei diesem bekannten Verfahren zur Vermeidung von Nebenreaktionen in der sauren Reaktionsgrenzschicht basische Elektrolyte zugegeben.

Die Elektrosynthese der 4-tert.-Butoxybenzaldehyddialkylacetale nach dieser Erfindung kann sowohl in einer geteilten als auch in einer ungeteilten Zelle durchgeführt werden. Als Elektrolyt dient hierbei eine Lösung von 4-tert.-Butoxytoluol in dem verwendeten Alkohol, die ein Leitsalz enthält. Die Leitsalze sollen im Elektrolyten löslich und unter den Versuchsbedingungen weitgehend stabil sein. Beispiele hierfür sind Fluoride, wie KF, Tetrafluoroborate wie $Et_4NBF_4$ und Perchlorate wie $Et_4NClO_4$. Als Anodenmaterialien werden beispielsweise Graphit oder Edelmetalle wie Platin eingesetzt. Stromdichte und Umsatz können in weiten Grenzen gewählt werden. Die Stromdichten betragen beispielsweise 1 bis 20 A/dm², die Elektrolyse wird z.B. mit 2 bis 5 F/Mol 4-tert.-Butoxytoluol und bei Temperaturen zwischen 15 und 60°C durchgeführt.

Die Zusammensetzung des Elektrolyten kann in weiten Grenzen gewählt werden. Die Elektrolytlösungen haben z.B. folgende Zusammensetzung:

4 bis 20 Gew.% 4-tert.-Butoxytoluol
70 bis 95 Gew.% Alkohol
0,4 bis 10 Gew.% Leitsalz.

Die Elektrolyseausträge werden zweckmässig destillativ aufgearbeitet. Überschüssiger Alkohol und gegebenenfalls noch vorhandenes Ausgangsmaterial werden von den Acetalen durch Destillation abgetrennt und können zur Elektrolyse zurückgeführt werden. Die 4-tert.-Butoxybenzaldehyddialkylacetale lassen sich, sofern erforderlich, durch Rektifikation weiter reinigen. Dass man die 4-tert.-Butoxybenzaldehyddialkylacetale nach dem erfindungsgemässen Verfahren, bei dem ein Zusatz von basischen Elektrolyten nicht erforderlich ist, so vorteilhaft herstellen kann, ist überraschend.

Die 4-tert.-Butoxybenzaldehyddialkylacetale eignen sich hervorragend für die Herstellung von 4-Hydroxybenzaldehyd, so dass diese Erfindung einen neuen vorteilhaften Zugang zu diesem wertvollen Vorprodukt für die Herstellung von halbsynthetischen Penicillinen eröffnet.

Für die Herstellung von 4-Hydroxybenzaldehyd sind mehrere Verfahren bekannt. So kann man 4-Hydroxybenzaldehyd z.B. herstellen durch:

1. Diazotierung von p-Aminobenzaldehyd und anschliessende Hydrolyse [s. Recueil des Travaux Chimiques des Pays-Bas 54 (1935) 97].

2. Kondensation von Phenol mit Formaldehyd in alkalischer Lösung und nachfolgende Behandlung mit dem Natriumsalz der 3-Nitrobenzolsulfonsäure (DE-PS 580 981).

3. Hydrierung von p-Hydroxybenzonitril [Helvetica Chimica Acta 19 (1936), 588].

4. Chlorierung von p-Tolylchlorformiat und alkalische Verseifung des Reaktionsproduktes (DE-OS 1 925 195).

5. Behandeln von Phenol mit Glyoxylsäure und Aufarbeitung des Reaktionsproduktes mit einer Quecksilberchlorid-Lösung (DE-PS 621 567).

6. Erhitzen von p-Methoxybenzaldehyd mit Pyridiniumhydrochlorid auf 200 bis 220°C [Ber. Dtsch. Chem. Ges. 74, 7 (1941), 1219].

7. Umsetzung von Phenol mit Trichloressigsäure und alkalische Verseifung der Reaktionsprodukte (J. Chem. Soc. 1933, 496).

8. Umsetzung von Phenol mit Chloroform in Gegenwart von β-Cyclodextrin (Die Pharmazie 1978, S. 467) und anschliessende Trennung vom ebenfalls entstehenden Salicylaldehyd, z.B. durch Wasserdampfdestillation.

Diese Verfahren haben die Nachteile, dass Isomerengemische entstehen (Verfahren 2, 8),

die Ausgangssubstanzen schwer zugänglich sind (Verfahren 1, 2, 3, 7) und sich bei ihrer technischen Durchführung erhebliche Umweltbelastungen ergeben (Verfahren 4, 5, 6, 7, 8).

Es wurde nun gefunden, dass man 4-Hydroxybenzaldehyd erheblich vorteilhafter herstellen kann, wenn man als Ausgangsstoffe die nach den erfindungsgemässen Verfahren erhältlichen 4-tert.-Butoxybenzaldehyddialkylacetale verwendet. So lässt sich 4-Hydroxybenzaldehyd auf einfache Weise dadurch herstellen, dass man die 4-tert.-Butoxybenzaldehyddialkylacetale mit Säuren behandelt. Man verwendet hierbei bevorzugt verdünnte wässrige Säuren. Obwohl es auch möglich ist, nicht verdünnte Säuren einzusetzen, bietet dies, abgesehen von der möglicherweise kürzeren Reaktionszeit, keine Vorteile, da die Ausbeuten dadurch nicht besser werden. Die Säurebehandlung führt man z.B. bei Temperaturen von 20 bis 140°C, vorzugsweise 60 bis 100°C durch. Dass sich die 4-tert.-Butoxybenzaldehyde so leicht in 4-Hydroxybenzaldehyd überführen lassen, ist sehr überraschend, da die Etherspaltung der entsprechenden n-Alkylether bekanntlich drastische Bedingungen erfordert.

Als Säuren können z.B. verwendet werden: Mineralsäuren wie Salzsäure, Phosphorsäure oder Schwefelsäure, aliphatische Carbonsäuren wie Ameisensäure, Essigsäure, Propionsäure oder Acrylsäure, Di- und Polycarbonsäuren wie Maleinsäure, Fumarsäure, Oxalsäure oder Butantetracarbonsäure, Hydroxycarbonsäuren wie Milchsäure oder Weinsäure, aromatische Carbonsäuren wie Benzoesäure, Phthalsäure, Mandelsäure oder p-Toluolsulfonsäure. Selbstverständlich können auch saure Ionenaustauscher verwendet werden.

Wenn der Methyl- und der tert.-Butylester der verwendeten Säure nicht flüchtig ist, z.B. bei Schwefelsäure, Oxalsäure oder bei Verwendung von Ionenaustauschern, reichen katalytische Mengen an Säure aus, vorzugsweise setzt man dann ca. 1 bis 10% bez. auf die 4-tert.-Butoxybenzaldehyddialkylacetale ein. Wenn die Ester leicht flüchtig sind, werden die 4-tert.-Butoxybenzaldehyddialkylacetale in einer Menge an Säure erhitzt, die mindestens dem stöchiometrisch notwendigen Verhältnis entspricht; da bei der Verseifung der Acetalgruppe zumindest ein Teil der eingesetzten flüchtigen Säure als Alkylester verloren geht, bedeutet dies, dass pro Mol Acetal mindestens 3 Mol der flüchtigen Säure eingesetzt werden. Zweckmässigerweise wird ein ca. 0,2 bis 3facher Überschuss an Säure verwandt, damit eine vollständige Umsetzung gewährleistet ist. Die Umsetzung ist in der Regel nach 10 min bis 3 Stunden beendet. Beim Abkühlen kristallisiert ein Teil des 4-Hydroxybenzaldehyds aus der Lösung aus, ein grosser Teil des Restes kann nach Einengen des Lösungsmittels isoliert werden.

Man kann bei der Herstellung des 4-Hydroxybenzaldehyds aus 4-tert.-Butoxybenzaldehyddialkylacetalen auch so verfahren, dass man in einem ersten Reaktionsschritt durch Umsetzung mit Wasser 4-tert.-Butoxybenzaldehyd herstellt, welches dann der Säurebehandlung unterworfen wird, wobei anstelle der mindestens 3fach molaren Menge an flüchtiger Säure nur eine mindestens molare Menge an Säure benötigt wird. Bei dieser zweistufigen Arbeitsweise wird der 4-tert.-Butoxybenzaldehyd in nahezu quantitativer Ausbeute erhalten.

In diesem Fall verfährt man zweckmässigerweise so, dass man die Säure in nicht verdünnter Form einsetzt und das Wasser erst nach beendeter Reaktion hinzusetzt, um die Löslichkeit des 4-Hydroxybenzaldehyds in der Säure zu verringern.

Beispiel 1

Herstellung von 4-tert.-Butoxy-benzaldehyd-dimethylacetal

| | |
|---|---|
| Apparatur: | ungeteilte Zelle mit 11 Elektroden |
| Anoden: | Graphit |
| Elektrolyt: | 475,8 g p-tert.-Butoxytoluol<br>2159 g Methanol<br>25 g KF |
| Kathoden: | Graphit |
| Temperatur: | 27 bis 31°C |
| Stromdichte: | 4,7 A/dm² |
| Elektrolyse mit: | 4,5 F/Mol p-tert.-Butoxytoluol |

Der Elektrolyt wird während der Elektrolyse über einen Wärmetauscher umgepumpt.

Aufarbeitung:

Nach Beendigung der Elektrolyse werden Methanol abdestilliert, KF (20 g) abfiltriert und der Rückstand bei 1 Torr und 50 bis 110°C fraktioniert destilliert. Hierbei erhält man neben 37,5 g unumgesetztem p-tert.-Butoxytoluol, 10,5 g p-tert.-Butoxybenzylmethyläther (kann zur Elektrolyse zurückgeführt werden) und 480,3 g p-tert.-Butoxybenzaldehyddimethylacetal. Dies entspricht einer Gesamtausbeute von 81,7%, die Stromausbeute beträgt 65,6%.

Beispiel 2

Herstellung von 4-tert.-Butoxy-benzaldehyddiäthylacetal

| | |
|---|---|
| Apparatur: | ungeteilte Zelle mit 11 Elektroden |
| Anoden: | Graphit |
| Elektrolyt: | 328 g p-tert.-Butoxy-toluol<br>2340 g Äthanol<br>15 g KF |
| Kathoden: | Graphit |
| Temperatur: | 25 bis 30°C |
| Stromdichte: | 1,2 A/dm² |
| Elektrolyse mit: | 5 F/Mol p-tert.-Butoxytoluol |

Der Elektrolyt wird während der Elektrolyse über einen Wärmetauscher umgepumpt.

Aufarbeitung:

Nach Beendigung der Elektrolyse werden Äthanol abdestilliert, KF (14 g) abfiltriert und der

Rückstand bei 1 bis 2 Torr und 60 bis 130°C fraktioniert destilliert. Hierbei erhält man neben 66,5 g unumgesetzten p-tert.-Butoxytoluol 37,2 g [(p-tert.-Butoxy)-benzyl]-äthyläther (kann zur Elektrolyse zurückgeführt werden) und 215,5 g 4-tert.-Butoxy-benzaldehyd-diäthylacetat. Dies entspricht einer Ausbeute von 60,2%.

Beispiel 3
Verwendung von 4-tert.-Butoxybenzaldehyddimethylacetal für die Herstellung von 4-Hydroxybenzaldehyd

a) 224 g 4-tert.-Butoxybenzaldehyddimethylacetal, 360 g Essigsäure und 150 g Wasser werden in einer Destillationsapparatur erhitzt. Bei einer Übergangstemperatur von 63 bis 100°C werden 450 g abdestilliert. Anschliessend wird noch ½ Stunde unter Rückfluss erhitzt.

Nach Zugabe von 300 g Wasser und Abkühlen auf −5°C können 103 g 4-Hydroxybenzaldehyd isolert werden. Von der Mutterlauge werden weitere 310 g abdestilliert, dann kühlt man den Rückstand auf −5°C ab. Es lassen sich weitere 14 g 4-Hydroxybenzaldehyd isolieren (Gesamtausbeute 96%).

b) 200 g 4-tert.-Butoxybenzaldehyddimethylacetal, 15 g Schwefelsäure sowie 800 ml Wasser werden in einer Rektifikationsapparatur unter Stickstoff erhitzt. Man destilliert ab, bis die Übergangstemperatur 95°C erreicht hat, und lässt gleichviel Wasser zutropfen wie Flüssigkeit abdestilliert (ca. 300 ml). Anschliessend lässt man noch 1 Stunde unter Rückfluss kochen. Beim Abkühlen auf Raumtemperatur fällt aus der Lösung reiner 4-Hydroxybenzaldehyd aus. Nach der üblichen Aufarbeitung kann man 98 g isolieren (Ausbeute 90,7%).

c) 400 g 4-tert.-Butoxybenzaldehyddimethylacetal, 50 g des unter der Bezeichnung DOWEX 50 WX8 im Handel erhältlichen Ionenaustauschers, der aus einem vernetzten kernsulfonierten Polystyrolharz besteht, sowie 1,6 l Wasser werden erhitzt. Weitere Verfahrensweise analog 3b. Die heisse Lösung wird vom Ionenaustauscher abdekantiert. Aus der erkalteten Lösung können 190 g 4-Hydroxybenzaldehyd isoliert werden (Ausbeute 88%).

d) 200 g 4-tert.-Butoxybenzaldehyddimethylacetal, 20 g Oxalsäure und 1 l Wasser werden wie unter Beispiel 3b angegeben, erhitzt. Man isoliert 102 g 4-Hydroxybenzaldehyd (Ausbeute 94,4%).

Beispiel 4
Verwendung von 4-tert.-Butoxybenzaldehyddimethylacetal zur Herstellung von 4-Hydroxybenzaldehyd unter Isolierung der Zwischenstufe

1028,3 g 4-tert.-Butoxybenzaldehyddimethylacetal werden in 750 g Wasser unter Rühren auf 95°C erwärmt. Während einer Stunde werden dann bei 95°C unter Rühren etwa 500 ml eines Gemisches aus Methanol und Wasser abdestilliert und gleichzeitig 500 g Frischwasser zugetropft. Anschliessend werden die Phasen getrennt. Die organische Phase wird bei 0,5 bis 1,5 Torr und 87 bis 107°C fraktioniert destilliert. Man erhält 761,7 g 4-tert.-Butoxybenzaldehyd. Dies entspricht einer Ausbeute von 93,2%.

a) 90 p-tert.-Butoxybenzaldehyd und 420 g 10%ige Salzsäure werden 15 min unter Rühren erhitzt. Beim Abkühlen auf 0°C kristallisiert 4-Hydroxybenzaldehyd aus; Gewicht nach dem Trocknen: 57 g (Schmp. 115 bis 116°C, Literatur: 117 bis 119°C); Ausbeute: 92% der Theorie.

b) 90 g p-tert.-Butoxybenzaldehyd, 70 g Essigsäure und 140 g Wasser werden 2 Stunden unter Rückfluss gekocht. Das Reaktionsgemisch wird am Rotationsverdampfer zur Trockne eingedampft. Es bleiben 60,5 g eines weissen Rückstandes zurück, der nach der NMR-Analyse aus reinem 4-Hydroxybenzaldehyd besteht (Ausbeute: 98% der Theorie).

**Patentansprüche**

1. Verfahren zur Herstellung von 4-tert.-Butoxybenzaldehyddialkylacetalen der Formel

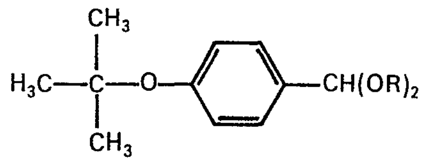

in der R eine Alkylgruppe mit 1 bis 8 C-Atomen bedeutet, dadurch gekennzeichnet, dass man 4-tert.-Butoxytoluol in Gegenwart eines Alkohols der Formel ROH, in der R die oben genannte Bedeutung hat, elektrochemisch oxidiert.

2. Verwendung der in Anspruch 1 gekennzeichneten 4-tert.-Butoxybenzaldehyddialkylacetale für die Herstellung von 4-Hydroxybenzaldehyd.

3. 4-tert.-Butoxybenzaldehyddimethylacetal.

**Claims**

1. A process for the preparation of a 4-tert.-butoxybenzaldehyde-dialkylacetal of the formula

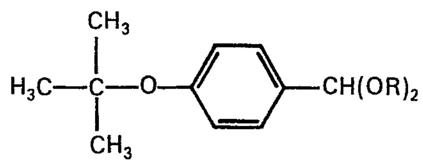

where R is alkyl of 1 to 8 carbon atoms, wherein 4-tert.-butoxytoluene is oxidized electrochemically in the presence of an alcohol of the formula ROH, where R has the above meaning.

2. Use of a 4-tert.-butoxybenzaldehyde-dialkylacetal as defined in claim 1 for the preparation

of 4-hydroxybenzaldehyde.

3. 4-tert.-butoxybenzaldehyde-dimethylacetal.

**Revendications**

1. Procédé de préparation de dialkylacétals du 4-tert.-butoxybenzaldéhyde de formule

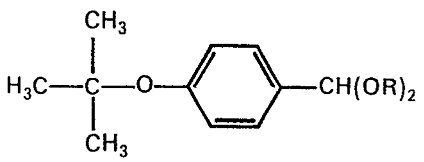

dans laquelle R représente un groupe alkyle en C1-C8, caractérisé en ce que l'on soumet à oxydation électrochimique le 4-tert.-butoxytoluène en présence d'un alcool de formule ROH dans laquelle R a la signification indiquée ci-dessus.

2. Utilisation des dialkylacétals du 4-tert.-butoxybenzaldéhyde caractérisés dans la revendication 1 dans la préparation du 4-hydroxybenzaldéhyde.

3. Le diméthylacétal du 4-tert.-butoxybenzaldéhyde.